# EUROPEAN PATENT SPECIFICATION

(11) **EP 0 548 535 B1**
(45) Date of publication and mention of the grant of the patent: **06.12.1995**
(21) Application number: 92119592.1
(22) Date of filing: 17.11.1992
(51) Int. Cl.: A61F 2/30, A61F 2/38

(54) **Endoprosthesis for the knee-joint**
Endoprothese für das Kniegelenk
Endoprothèse pour l'articulation du genou

(30) Priority: 20.12.1991 DE 9115810 U
(43) Date of publication of application: 30.06.1993
(73) Proprietor: Howmedica GmbH, D-24232 Schönkirchen (DE)
(72) Inventor: Kotz, Rainer, Prof. Dr., A-1010 Vienna (AT); Windhager, Reinhard, Dr., A-1190 Vienna (AT)
(74) Representative: Patentanwälte Hauck, Graalfs, Wehnert, Döring, Siemons

(56) References cited:
- EP-A- 0 290 767
- EP-A- 0 350 337
- DE-U- 9 115 810
- US-A- 2 700 772
- US-A- 4 502 160

## Description

The present invention refers to an endoprosthesis for the knee-joint according to the preamble of claim 1.

For patients who are still growing and who carry a full endoprosthesis for the knee-joint for example, an adaptation to the growth in length is necessary. German petty patent DE-GM 87 06 999 discloses a so-called growth prosthesis comprising a pair of telescopically coacting rod members defining a bone replacement part, wherein a spindle and a spindle nut provide for a relative adjustment of the rod members. The spindle of the known prosthesis is coupled to a bevel gear of which a gear wheel includes a drive pin which may be actuated from outside the prosthesis. Preferrably the drive pin is located in an area of the joint where the thickness of the soft tissue is relatively thin so that the bevel gear becomes accessible by a mere prick incision. It therefore needs a local an-esthesia only to adjust the prosthesis in length. Nevertheless an operation, although minor, is necessary resulting in a certain stress and risk the patient is subjected to.

It is the object of the present invention to provide a length-adjustable prosthesis for the knee joint including an adjacent bone portion wherein the length may be adjusted post-operative without operation.

According to the invention the object is solved by the features of claim 1.

According to the invention an index drive is provided in the prosthesis which is actuated by the tibial or femoral joint member in a more or less extreme bending position of the joint. An indexing member of the indexing drive directly actuates the member to be moved, i.e. a threaded spindle in steps. The indexing member is actuated in an extreme bending position which is limited by the geometry of the knee-prosthesis to obtain a rotation of the spindle. According to a preferred embodiment of the invention a ratchet mechanism is provided in which typically an indexing pawl engages the teeth of a wheel, whereas a locking pawl locks the indexing wheel when the driving wheel performs its return stroke.

A number of different structural embodiments is suitable to build a ratchet mechanism. According to an embodiment of the invention, the indexing member is defined by a pin which is slidably supported in the femoral or tibial joint member which pin coacts with the toothing of a gear wheel. When the indexing pin is actuated by bending the joint the gear wheel is driven at a small rotational angle. A repeated bending and stretching thus results in a corresponding stepwise turning of the threaded spindle.

When the indexing pin coacting with the tooth flanks of the gear wheel provides for an indexing of less than the circular tooth pitch, a spring loaded locking pawl cooperating with the toothing of the pinion is provided to rotate the pinion around half the pitch when the indexing pin is out of engagement with the toothing. Accordingly a rotation of half the pitch is performed by the pin and the remaining rotation by the locking pawl.

According to a further embodiment of the invention the indexing pin is biased by a spring to disengage the toothing in the normal case.

The invention is described in detail with reference to the drawings.
- Fig. 1: shows a side view, partly in section, of a prosthesis according to the invention,
- Fig. 2: shows a front view of an indexing pinion of the prosthesis of Fig. 1,
- Fig. 3: shows a side view portion of the pinion of Fig. 1 in the direction of arrow 3,
- Fig. 4: shows a view illustrating a locking pawl coacting with the pinion of the prosthesis of Fig. 1.

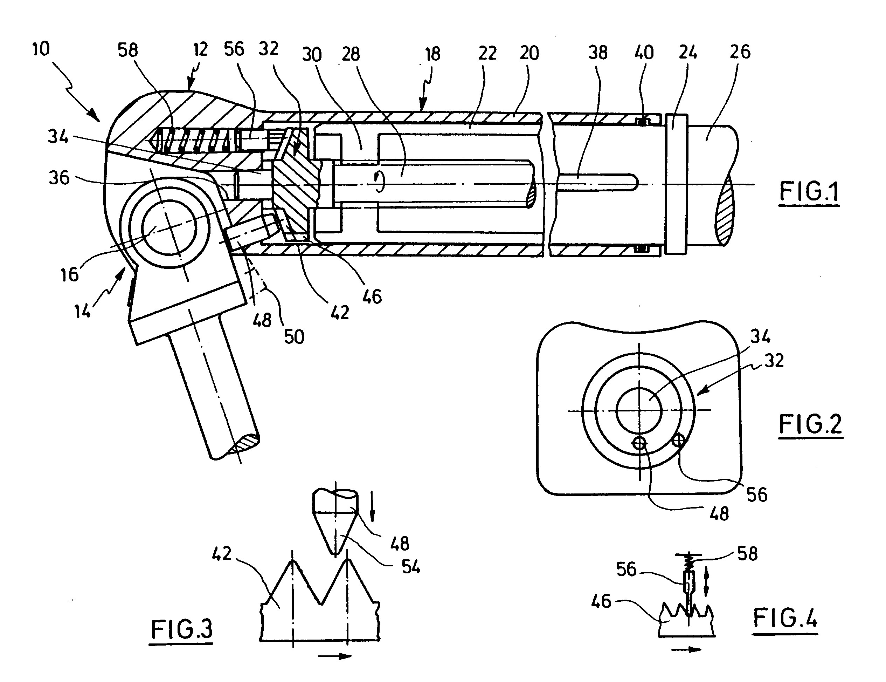

The endoprosthesis shown in Fig. 1 comprises a prosthetic knee joint 10 of the type of a hinge joint including a femoral joint portion 12 and a tibial joint portion 14. The joing portions 12, 14 are pivotably arranged about the rotating pin 16. The tibial joint portion 14 comprises means not shown for attaching to the human tibia. A bone replacement part 18 is attached to the femoral joint portion 12. The replacement part comprises a tube 20 integrally connected to the femoral portion 12 in which tube an inner-cylindrical part 22 is telescopically slidingly received. A displacement of the inner part 22 into the outer part is limited by a radial flange 24. The side of the flange 24 opposite the part 20 is connected to a solid shaft 26 comprising means (not shown) for attaching to the femur. A suitable attachment is shown in German petty patent DE-GM 87 06999 for example.

The inner telescopic part 22 is formed partly hollow and accomodates a threaded spindle 28 cooperating with a spindle nut 30 which is formed by a cross-wall of the inner telescopic part 22. The spindle 28 integrally carries an indexing pinion 32 having a front end comprising a pin 34 which is rotatably supported in a bore 36 of the femoral part 12. When the spindle 28 is rotated, the telescopic part 22 relatively moves with respect to the telescopic part 20 to obtain a change in length. The telescopic part 22 is non-rotatably fixed as indicated at 38. Both the telescopic parts are sealed with respect to each other as indicated by the seal 40. The indexing pin comprises a first bevelled toothing 42 and a second toothing 46 (see Fig. 4 as well). The femoral part 12 slidingly supports an indexing pin 48 which can be biased by a spring not shown away from the indexing pinion 32. The indexing pin 48 projects over the femoral part 42, wherein the outwardly directed motion may be limited by means not shown. When the tibial part 14 is further bent with respect to the femoral part, as indicated in dash-dotted lines 50, the tibial part 14 urges the indexing pin 48 towards the pinion 32. Accordingly, the conical tip 54 of the pin 48 engages an adjacent flank of the toothing 42 to rotate the pinion 32 about half a pitch of the toothing 42. A locking pawl 56 in shape of a pin urged by pressure spring 58 towards the pinion 32 cooperates with the toothing 46. When the pinion 32 is rotated as mentioned before, the pawl 56 disengages the toothing. As the indexing pin 48 rotates the pinion such that the toothing 46 is rotated about half the pitch or somewhat more, the locking pawl 46 can enter the next tooth gap to further rotate the pinion about a certain amount. Repeating the steps referred to, the spindle is rotated, further resulting in the desired change of length by moving the inner telescopic part 22 out of the outer part.

The artisan realises that the indexing mechanism performs a length adaptation of the prothesis eliminating the need of a surgical operation.

Fig. 4 shows that the profile of the flanks of the toothing 46 is different. When the locking pin 56 moves out against the force of the spring 58, the pin 56 slides along a profile which degressively slopes from the gear bottom whereas the profile on the opposite side of the tooth forms a straight inclined ramp.

## Claims

1. Endoprothesis for the knee joint and adjacent bone parts comprising a femoral (12) and a tibial joint part (14), a bone replacement part (18) to be attached to the femoral or, respectively tibial joint part which replacement part comprises a pair of telescopically cooperating rod members (20,22) which are adjusted with respect to each other by a threaded spindle (28) and a spindle nut (30), and comprising a pinion on the spindle to be actuated by a rotating means, characterized in that an indexing mechanism is provided comprising an indexing member (48) which is actuated by the tibial or femoral joint part (12, 14) in an extreme bending position of the joint.

2. The endoprothesis of claim 1, characterized in that a ratchet mechanism (32, 48, 56) is provided.

3. The endoprothesis of claim 2, characterized in that the indexing member is defined by a pin (48) which is slidably supported in the femoral or tibial joint part (12, 14) which pin cooperates with the toothing (42) of the pinion (32).

4. The endoprothesis of claim 3, characterized in that a spring-loaded locking pawl (56) is provided coacting with a toothing (46) of the pinion (32) which pawl rotates the pinion about half the pitch when the indexing pin (48) disengages the toothing (42).

5. The endoprothesis of claim 3 or 4, characterized in that the indexing pin is biased by a spring away from the toothing.

6. The endoprothesis of claim 4 or 5, characterized in that the tooth profile along which the locking pawl (56) is moved out of the toothing (46), when the locking pin (48) further rotates the pinion (32), degressively slopes upwardly from the tooth bottom.

## Patentansprüche

1. Endoprothese für das Kniegelenk und angrenzende Knochenteile mit einem Oberschenkel- (12) und einem Schienbeingelenkteil (14), einem Knochenersatzteil (18) zur Anbringung an dem Oberschenkel- oder dem Schienbeingelenkteil, wobei das Knochenersatzteil ein Paar von ineinanderschiebbar zusammenwirkenden Stabteilen (20, 22) aufweist, die in bezug aufeinander durch eine mit einem Gewinde versehene Welle (28) und eine Wellenmutter (30) eingestellt werden, und mit einem durch ein Rotationsmittel bewegten Ritzel auf der Welle, dadurch gekennzeichnet, daß ein Schaltmechanismus vorgesehen ist, der ein Schaltteil (48) aufweist, das durch das Schienbein- oder das Oberschenkelgelenkteil (12, 14) in einer extremen Beugeposition des Gelenks bewegt wird.

2. Endoprothese nach Anspruch 1, dadurch gekennzeichnet, daß ein Sperrmechanismus (32, 48, 56) vorgesehen ist.

3. Endoprothese nach Anspruch 2, dadurch gekennzeichnet, daß das Schaltteil durch einen Bolzen (48) gegeben ist, der verschiebbar in dem Oberschenkel- oder Schienbeingelenkteil (12, 14) gehalten ist, wobei der Bolzen mit der Zahnung (42) des Ritzels (32) zusammenwirkt.

4. Endoprothese nach Anspruch 3, dadurch gekennzeichnet, daß eine federbelastete Sperrklinke (56) vorgesehen ist, die mit einer Zahnung (46) des Ritzels (32) zusammenwirkt, wobei die Sperrklinke das Ritzel um die halbe Zahnteilung dreht, wenn der Schaltbolzen (48) die Zahnung (42) verläßt.

5. Endoprothese nach Anspruch 3 oder 4, dadurch gekennzeichnet, daß der Schaltbolzen durch eine Feder von der Zahnung weg vorgespannt ist.

6. Endoprothese nach Anspruch 4 oder 5, dadurch gekennzeichnet, daß das Zahnprofil, entlang dem die Sperrklinke (56) aus der Zahnung (46) bewegt wird, wenn der Sperrbolzen (48) das Ritzel (32) weiterdreht, abnehmend von dem Zahnboden ansteigt.

## Revendications

1. Endoprothèse pour l'articulation du genou et des parties d'os adjacentes, comprenant des pièces fémorale (12) et tibiale (14) d'articulation, une pièce (18) de remplacement de l'os destinée à être fixée à la pièce fémorale ou, respectivement tibiale de l'articulation, laquelle pièce de remplacement comprend deux éléments à tige, (20, 22) en coopération télescopique, qui sont ajustés l'un par rapport à l'autre par une broche filetée (28) et un écrou (30) de broche, et comportant un pignon sur la broche destiné à être actionné par un moyen de rotation, caractérisée en ce qu'un mécanisme d'indexage est prévu, lequel comporte un élément d'indexage (48) qui est actionné par la pièce tibiale Ou fémorale (12, 14) d'articulation dans une position de flexion extrême de l'articulation.

2. Endoprothèse selon la revendication 1, caractérisée en ce qu'un mécanisme d'encliquetage (32, 48, 56) est prévu.

3. Endoprothèse selon la revendication 2, caractérisée en ce que l'élément d'indexage est défini par un ergot (48) qui est supporté de façon à pouvoir coulisser dans la pièce fémorale ou tibiale (12, 14) de l'articulation, lequel ergot coopère avec la denture (40) du pignon (32).

4. Endoprothèse selon la revendication 3, caractérisée en ce qu'un cliquet (56) de verrouillage, rappelé par ressort, est prévu de façon à coopérer avec une denture (46) du pignon (32), lequel cliquet fait tourner le pignon d'environ la moitié du pas lorsque l'ergot (48) d'indexage se dégage de la denture (42).

5. Endoprothèse selon la revendication 3 ou 4, caractérisée en ce que l'ergot d'indexage est rappelé par un ressort à l'écart de la denture.

6. Endoprothèse selon la revendication 4 ou 5, caractérisée en que le profil des dents le long desquelles le cliquet (56) de verrouillage se dégage de la denture (46), lorsque l'ergot (48) de verrouillage fait tourner davantage le pignon (32), est en pente dégressive vers le haut à partir du fond d'entre-dents.
